(19) Europäisches Patentamt
European Patent Office
Office européen des brevets

(11) Veröffentlichungsnummer: **0 051 198**
**B1**

# (12) EUROPÄISCHE PATENTSCHRIFT

(45) Veröffentlichungstag der Patentschrift: **25.01.84**

(51) Int. Cl.³: **C 07 C 147/00**, A 01 N 41/10

(21) Anmeldenummer: **81108489.6**

(22) Anmeldetag: **19.10.81**

## (54) Verfahren zur Herstellung von 2-Halogen-3-sulfonyl-acrylnitrilen und deren Verwendung.

(30) Priorität: **31.10.80 DE 3041154**

(43) Veröffentlichungstag der Anmeldung: **12.05.82 Patentblatt 82/19**

(45) Bekanntmachung des Hinweises auf die Patenterteilung: **25.01.84 Patentblatt 84/4**

(84) Benannte Vertragsstaaten: **BE CH DE FR GB IT LI NL SE**

(56) Entgegenhaltungen:
**FR - A - 2 296 623**

**METHODEN DER ORGANISCHEN CHEMIE, Band V/1b, Houben-Weyl, 1972, Georg Thieme Verlag Stuttgart, DE**

(73) Patentinhaber: **BAYER AG, Zentralbereich Patente, Marken und Lizenzen, D-5090 Leverkusen 1, Bayerwerk (DE)**

(72) Erfinder: **Oeckl, Siegfried, Dr., Andreas-Gryphius-Strasse 9, D-5000 Köln 80 (DE)**
Erfinder: **Zahl, Gero, Dr., Andreas-Gryphius-Strasse 7, D-5000 Köln 80 (DE)**
Erfinder: **Radt, Walter, Dr., Bethelstrasse 12, D-4015 Krefeld (DE)**

**EP 0 051 198 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2-Halogen-3-sulfonyl-acrylnitrilen und deren Verwendung.

Die Erfindung betrifft ein Verfahren zur Herstellung von 2-Halogen-3-sulfonyl-acrylnitrilen.

Es ist aus der DE-OS 2500265 bekannt, dass man aus 2,2-Dihalogen-3-sulfonyl-propionitrilen durch Abspaltung von Halogenwasserstoff mit Basen, wie Triethylamin, Alkalihydroxiden, Alkalialkoholaten, oder Alkalisalzen von Carbonsäuren 2-Halogen-3-sulfonyl-acrylnitrile erhält.

Es wurde ein Verfahren zur Herstellung von 2-Halogen-3-sulfonyl-acrylnitrilen der Formel

$$R^1-SO_2-CH=CX^1-CN, \quad (I)$$

worin
$R^1$ gegebenenfalls substituiertes Aryl, Aralkyl, Alkyl oder Cycloalkyl und
$X^1$ Halogen bedeutet,
gefunden, das dadurch gekennzeichnet ist, dass man 2,2-Dihalogen-3-sulfonyl-propionitrile der Formel

$$R^1-SO_2-CH_2-CX^1_2-CN, \quad (II)$$

worin
$R^1$ und $X^1$ die oben angegebene Bedeutung haben,
gegebenenfalls in Gegenwart eines organischen Lösungsmittels und gegebenenfalls bei erhöhter Temperatur mit einem Überschuss an Wasser behandelt.

Als Aryl seien aromatische Reste mit 6 bis 18 Kohlenstoffatomen genannt, die linear miteinander verknüpft oder kondensiert sein können. Beispielsweise seien Phenyl, Biphenyl, Naphthyl genannt. Bevorzugt sei Phenyl genannt.

Als Aralkyl seien Reste mit 7 bis 14 Kohlenstoffatomen genannt, wobei der aromatische Teil des Aralkyls bis zu 10 Kohlenstoffatome und der aliphatische Teil bis zu 6 Kohlenstoffatome enthalten kann. Beispielsweise seien Ethylphenyl, Benzyl, Naphthylmethyl genannt. Bevorzugt sei Benzyl genannt.

Als Alkyl seien geradkettige oder verzweigte Kohlenwasserstoffreste mit 1 bis 12 Kohlenstoffatomen, bevorzugt Niederalkylreste mit 1 bis 6 Kohlenstoffatomen, genannt. Beispielsweise seien Methyl, Ethyl, Propyl, Isopropyl, Butyl, Isobutyl, Pentyl, Isopentyl, Hexyl, Isohexyl, Octyl, Isooctyl, Undecyl, genannt. Bevorzugt seien Methyl und Ethyl genannt.

Als Cycloalkyl seien Kohlenwasserstoffreste mit 6 bis 17 Kohlenstoffatomen genannt, beispielsweise Cyclopentyl, Cyclohexyl, der Dekalinrest.

Als Halogen seien Fluor, Chlor, Brom und Jod, bevorzugt Chlor und Brom, besonders bevorzugt Chlor, genannt.

Die Reste können mit weiteren Resten substituiert sein, die sich unter den erfindungsgemässen Bedingungen nicht verändern. Beispielsweise seien Nitrogruppen, Cyanogruppen, Alkoxygruppen mit 1 bis 4 Kohlenstoffatomen, wie Methoxy, Ethoxy, Propoxy, iso-Propoxy, Butoxy, iso-Butoxy, Halogene wie Fluor, Chlor, Brom und Jod genannt.

Die im erfindungsgemässen Verfahren eingesetzten 2,2-Dihalogen-3-sulfonyl-propionitrile sind aus der US-PS 3437685 und DE-OS 2500265 bekannt. So können beispielsweise durch Umsetzung von 3-Sulfonylpropionitrilen mit Chlor oder Brom, 2,2-Dichlor- bzw. 2,2-Dibrom-3-sulfonyl-propionitrile hergestellt werden.

Die bei dieser Umsetzung verwendeten 3-Sulfonyl-propionitrile sind aus Roczniki Chemii 30, 243 (1956) bekannt. Sie werden z.B. durch Umsetzung von Sulfinsäuren oder Sulfinaten mit Acrylnitril im wässrigen Medium erhalten.

Bevorzugt geeignete 2,2-Dihalogen-3-sulfonyl-propionitrile für das erfindungsgemässe Verfahren sind Verbindungen der Formel

$$R^2-SO_2-CH_2-CX^2_2-CN, \quad (III)$$

worin
$R^2$ gegebenenfalls substituiertes Phenyl, Naphthyl, Benzyl, Naphthylmethyl, geradkettiges oder verzweigtes Niederalkyl wie Methyl, Ethyl, Propyl, iso-Propyl, Butyl, iso-Butyl, Pentyl, iso-Pentyl, Hexyl, iso-Hexyl, Cycloalkyl wie Cyclopentyl und Cyclohexyl und
$X^2$ Chlor oder Brom,
bedeuten.

Besonders bevorzugt geeignet sind für das erfindungsgemässe Verfahren 2,2-Dihalogen-3-sulfonyl-propionitrile der Formel

$$R^3-SO_2-CH_2-CX^3_2-CN, \quad (IV)$$

worin
$R^3$ Phenyl, Benzyl, Methyl, Ethyl, Cyclohexyl und
$X^3$ Chlor,
bedeuten.

Beispielsweise seien die folgenden 2,2-Dihalogen-3-sulfonyl-propionitrile genannt:

2,2-Dichlor-3-phenyl-sulfonyl-propionitril
2,2-Dichlor-3-benzyl-sulfonyl-propionitril
2,2-Dichlor-3-methyl-sulfonyl-propionitril
2,2-Dichlor-3-(3-chlor)-phenyl-sulfonyl-propionitril
2,2-Dichlor-3-(3,4-dichlor)-phenyl-sulfonyl-propionitril
2,2-Dichlor-3-(4-methyl)-phenyl-sulfonyl-propionitril
2,2-Dibrom-3-phenyl-sulfonyl-propionitril
2,2-Dibrom-3-benzyl-sulfonyl-propionitril
2,2-Dibrom-3-methyl-sulfonyl-propionitril
2,2-Dibrom-3-iso-propyl-sulfonyl-propionitril
2,2-Dichrom-3-cyclohexyl-sulfonyl-propionitril
2,2-Dibrom-3-cyclopentyl-sulfonyl-propionitril

Nach dem erfindungsgemässen Verfahren wird stets mit einem Überschuss an Wasser behandelt. Das Molverhältnis von 2,2-Dihalogen-3-sulfonylpropionitrilen zu Wasser bewegt sich im

allgemeinen in einem Bereich von 1:5 bis 1:550.

In einer bevorzugten Ausführungsform des erfindungsgemässen Verfahrens wird die Behandlung mit Wasser ohne organische Lösungsmittel durchgeführt. Im allgemeinen setzt man in diesem Fall 5 bis 100 Mol, bevorzugt 10 bis 50 Mol, besonders bevorzugt 15 bis 30 Mol, Wasser pro Mol 2,2-Dihalogen-3-sulfonyl-propionitril ein.

Die Menge an Wasser beträgt beispielsweise im Falle der Durchführung der Behandlung in Gegenwart eines organischen Lösungsmittels 10 bis 530 Mol, bevorzugt 15 bis 160 Mol, besonders bevorzugt 25 bis 100 Mol pro Mol 2,2-Dihalogen-3-sulfonyl-propionitril.

Als organische Lösungsmittel seien beispielsweise genannt:

Aliphatische und aromatische Kohlenwasserstoffe, wie Paraffine, Cyclohexan, Benzol und Toluol, Alkylbenzole, wie Xylole und Cumol, Halogenkohlenwasserstoffe, wie Dichlormethan, Chloroform, Dichlorethan, Tri- und Tetrachlorethylen, Ether, wie Diethylether, Methyl-tert.-butylether, Tetrahydrofuran und Dioxan, Ketone, wie Aceton, Methyl-ethylketon, Methyl-isobutylketon und Cyclohexanon, Ester, wie Ethyl- und Butylacetat, Alkohole, wie Methanol, Ethanol und Isopropanol, Glykole, wie Ethylenglykol, Glykolether und -ester, wie Glykolmonomethylether.

Selbstverständlich ist es möglich, Mischungen der Lösungsmittel zu verwenden. Es kann dabei ein homogenes Gemisch oder ein Mehrphasengemisch entstehen.

Gegebenenfalls werden pro Mol 2,2-Dihalogen-3-sulfonyl-propionitril im allgemeinen 100 bis 1000 ml, bevorzugt 250 bis 600 ml organisches Lösungsmittel verwendet.

Das erfindungsgemässe Verfahren wird im allgemeinen im Temperaturbereich von 20 bis 130°C, bevorzugt von 50 bis 120°C, durchgeführt. Bei der Durchführung des erfindungsgemässen Verfahrens ohne organisches Lösungsmittel mit einem Überschuss an Wasser wird die Umsetzung bevorzugt im Siedebereich des Wassers bei Normaldruck durchgeführt.

Das erfindungsgemässe Verfahren kann bei Unter-, Normal- oder Überdruck, beispielsweise von 0,5 bis 3 bar, durchgeführt werden. Bevorzugt wird unter Normaldruck gearbeitet.

Die Reaktionszeit des erfindungsgemässen Verfahrens liegt im allgemeinen bei etwa 8 bis 20 Stunden. Wird im Siedebereich des Wassers bei Normaldruck gearbeitet, kann die Reaktionszeit auf 5 bis 12 Stunden verkürzt werden. Das Ende der Reaktion kann durch analytische Methoden, beispielsweise Dünnschicht- oder Hochdruckflüssigkeits-Chromatographie festgestellt werden.

In einer bevorzugten Ausführungsform des erfindungsgemässen Verfahrens stellt man die 2,2-Dihalogen-3-sulfonyl-propionitrile durch Halogenierung von 3-Sulfonyl-propionitrilen her. Die 3-Sulfonyl-propionitrile werden bevorzugt durch Umsetzung von Sulfinaten mit Acrylnitril erhalten.

Im Rahmen dieser bevorzugten Ausführungsform erhält man die 2,2-Dihalogen-3-sulfonyl-propionitrile, indem man 3-Sulfonyl-propionitrile der Formel

$$R^1\text{-}SO_2CH_2CH_2CN, \qquad (V)$$

worin

$R^1$ die oben angegebene Bedeutung hat,

mit einem Halogenierungsmittel, wie einem Sulfurylhalogenid, einem Phosphorpentahalogenid oder einem Halogen, gegebenenfalls in einem Lösungsmittel bei einer Temperatur von 30 bis 130°C umsetzt.

Bevorzugt wird als Halogenierungsmittel Sulfurylchlorid, Phosphorpentachlorid, Chlor oder Brom, ganz besonders bevorzugt Chlor, verwendet.

Im allgemeinen werden 2 bis 4 Mol Halogenierungsmittel pro Mol 3-Sulfonyl-propionitril verwendet. Bevorzugt werden 2 Mol bis 3 Mol Halogenierungsmittel verwendet.

Das Halogenierungsmittel wird bevorzugt in dem Masse zugegeben, wie es mit dem 3-Sulfonyl-propionitril reagiert und dass keine Anreicherung an Halogenierungsmittel im Reaktionsgemisch eintritt.

Um die Bildung von Nebenprodukten zurückzudrängen, kann es bei der Durchführung der Halogenierung von Vorteil sein, die Umsetzung abzubrechen, wenn noch nicht alles eingesetzte 3-Sulfonyl-propionitril verbraucht ist. So kann man beispielsweise vorteilhaft die Umsetzung abbrechen, wenn ein Umsatz von 30 bis 95% erreicht ist.

Die Halogenierung kann ohne Lösungsmittel oder in einem für Halogenierungen üblichen Lösungsmittel, beispielsweise einem Halogenkohlenwasserstoff wie Dichlormethan, Chloroform, Dichlorethan, Tri- oder Tetrachlorethylen, durchgeführt werden.

Besonders bevorzugt werden im erfindungsgemässen Verfahren die 2,2-Dichlor-3-sulfonyl-propionitrile hergestellt, indem man die Chlorierung mit Chlor im Temperaturbereich von 80 bis 120°C ohne Lösungsmittel durchführt.

Bei der bevorzugten Herstellung der 2,2-Dihalogen-3-sulfonyl-propionitrile erhält man als Nebenprodukt (bis zu etwa 10 Gew.-%) 2,3-Dihalogen-3-sulfonyl-acrylnitrile der Formel

$$R^1\text{-}SO_2CX^1{=}CX^1\text{-}CN, \qquad (VI)$$

worin

$R^1$ und $X^1$ die oben angegebene Bedeutung haben.

Die so erhaltenen 2,2- und 2,3-Dihalogen-3-sulfonyl-acrylnitrile können ohne weitere Reinigung in das erfindungsgemässe Verfahren eingesetzt werden. Selbstverständlich ist es aber auch möglich, eine der üblichen Aufarbeitungsmethoden, beispielsweise Kristallisation oder Destillation, der Halogenierung anzuschliessen. Dabei können die 2,2-Dihalogen-3-sulfonyl-propionitrile und die 2,3-Dihalogen-3-sulfonyl-acrylnitrile gereinigt und/oder getrennt werden.

Es ist im allgemeinen zweckmässig, das Umsetzungsgemisch nicht zu trennen, sondern als Gemisch weiterzuverwenden.

Die bei der Halogenierung eingesetzten 3-Sulfonyl-propionitrile können durch Umsetzung von Sulfinaten der Formel

$(R^1\text{-}SO_2)_nM,$ (VII)

worin
$R^1$ die oben angegebene Bedeutung hat,
M für Wasserstoff, Ammonium oder ein Metall, steht und
n die Wertigkeit von M angibt,
in Wasser, gegebenenfalls in Gegenwart eines organischen Lösungsmittels, mit Acrylnitril unter gleichzeitiger Zugabe von Säure, erhalten werden.

Metalle des Restes M sind beispielsweise Alkalimetalle, wie Natrium oder Kalium, Erdalkalimetalle, wie Magnesium oder Calcium, oder zweiwertige Übergangsmetalle, wie Zink.

Als organische Lösungsmittel können solche verwendet werden, die unter den Reaktionsbedingungen inert sind. Beispielsweise können die gleichen Lösungsmittel verwendet werden, die bei der erfindungsgemässen Umsetzung der 2,2-Dihalogen-3-sulfonyl-propionitrile mit Wasser als Lösungsmittel zugesetzt werden können.

Selbstverständlich können auch Mischungen dieser Lösungsmittel untereinander verwendet werden, wobei ein homogenes Gemisch oder ein Mehrphasengemisch entstehen kann.

Bevorzugt wird ohne organisches Lösungsmittel in Wasser gearbeitet.

Die Sulfinate können vollständig gelöst oder teilweise gelöst vorliegen. Zur Erzielung besserer Raum/Zeit-Ausbeuten setzt man die Sulfinate in teilweise gelöster Form ein.

Die Umsetzung der Sulfinate mit dem Acrylnitril kann im Temperaturbereich von 0 bis 130°C durchgeführt werden. Bevorzugt wird bei 50 bis 120°C, besonders bevorzugt bei 90 bis 110°C gearbeitet.

Bei der Umsetzung der Sulfinate mit Acrylnitril kann durch kontinuierliche Zugabe von Säuren ein etwa konstanter pH-Wert eingehalten werden. Bevorzugt wird ein pH-Wert von 3 bis 8, besonders bevorzugt von 6 bis 7, eingehalten.

Als Säuren können im allgemeinen organische und anorganische Brönstedt-Säuren verwendet werden.

Als organische Brönstedt-Säuren seien genannt: Alkansäuren, wie beispielsweise Essig-, Propion-, Buttersäure, Dicarbonsäuren wie Oxalsäure, Malonsäure oder Bernsteinsäure, aromatische Carbonsäuren, wie Benzoesäure und Salicylsäure, Sulfonsäuren, wie Methan-, Ethan-, Benzol- oder Toluolsulfonsäuren.

Als anorganische Brönstedt-Säuren seien genannt: Wässrige Lösungen von Schwefelsäure, Halogenwasserstoffsäuren wie Salzsäure, Phosphorsäure, schweflige Säure oder Kohlendioxid.

Bevorzugt werden die Brönstedt-Säuren im Konzentrationsbereich von 20 bis 70% eingesetzt. Besonders bevorzugt wird 40 bis 60%ige Schwefelsäure eingesetzt.

Im allgemeinen legt man die Sulfinate vor und gibt das Acrylnitril in dem Masse zu, wie es sich umsetzt. Gleichzeitig mit dem Acrylnitril wird die Säure so zugegeben, dass ein erfindungsgemässer konstanter pH-Bereich eingehalten wird.

Die Aufarbeitung des Umsetzungsgemisches kann beispielsweise durch Phasentrennung, durch Extraktion oder durch Kristallisation erfolgen. Bevorzugt wird mit Hilfe einer Phasentrennung aufgearbeitet. Die Phasentrennung kann mit dem durch Temperaturerhöhung verflüssigten Produkt selbst oder mit Hilfe eines vor, während oder nach der Umsetzung zugesetzten organischen Lösungsmittels erfolgen. Wird beispielsweise Wasser als Umsetzungsmedium verwendet, können mit Wasser nicht oder nur schwer mischbare organische Lösungsmittel eingesetzt werden. Vorzugsweise können als organische Lösungsmittel aromatische Kohlenwasserstoffe wie Toluol oder Xylole eingesetzt werden.

Wenn man die Umsetzung in Wasser als Lösungsmittel, beispielsweise mit sehr hoher Konzentration an Sulfinaten und Acrylnitril durchführt, kann man neben den gegebenenfalls beiden flüssigen Phasen noch eine feste, durch ausgefallene Salze erhalten. In diesem Fall kann man durch Nachwaschen, beispielsweise mit Wasser, diese Salze entfernen.

Die Umsetzungsprodukte werden mit hoher Reinheit erhalten, so dass sie ohne weitere Reinigungsschritte für die Halogenierung verwendet werden können. Es ist aber selbstverständlich möglich, die Umsetzungsprodukte vor der Weiterverwendung, beispielsweise durch Destillation oder Kristallisation, weitergehend zu reinigen.

In einer ganz besonders bevorzugten Ausführungsform des erfindungsgemässen Verfahrens können die 2-Halogen-3-sulfonyl-acrylnitrile ausgehend von den Sulfinaten mit Acrylnitril ohne Isolierung von Zwischenprodukten in einer Eintopfreaktion hergestellt werden, die dadurch gekennzeichnet ist, dass in einem ersten Reaktionsschritt die Sulfinate mit Acrylnitril umgesetzt, in einem zweiten Reaktionsschritt die erhaltenen 3-Sulfonyl-propionitrile halogeniert und die erhaltenen 2,2-Dihalogen-3-sulfonyl-propionitrile mit Wasser behandelt werden. Die so erhaltenen 2-Halogen-3-sulfonyl-acrylnitrile können 2,3-Dihalogen-3-sulfonyl-acrylnitrile enthalten.

Es ist überraschend, dass bei der erfindungsgemässen Umsetzung der 2,2-Dihalogen-3-sulfonyl-propionitrile mit Wasser eine Abspaltung von Halogenwasserstoff eintritt und die Nitrilgruppen unverändert bleiben. Es war zu erwarten, dass die Nitrilgruppen durch das Wasser in Gegenwart des entstehenden Halogenwasserstoffs verseift werden.

Bei der bevorzugten Herstellung der 2,2-Dihalogen-3-sulfonyl-propionitrile ist es überraschend, dass sie mit hoher Selektivität durch Halogenierung der 3-Sulfonyl-propionitrile hergestellt werden.

Bei der bevorzugten Herstellung der 3-Sulfonyl-propionitrile ist es überraschend, dass sie bei der Umsetzung der Sulfinate mit Acrylnitrilen bei Temperaturen von 80 bis 120°C in hoher Ausbeute erhalten werden.

Nach dem erfindungsgemässen Verfahren erhält man 2-Halogen-3-sulfonyl-acrylnitrile, die, wie aus der DE-OS 25 00 265 bekannt ist, mikrobizide Wirkstoffe sind.

Bevorzugt können die Gemische aus 2-Halogen-3-sulfonyl-acrylnitrilen und dem Nebenprodukt 2,3-Dihalogen-3-sulfonyl-acrylnitrilen in mikrobiziden Mitteln für den Schutz technischer Materialien verwendet werden. Diese Gemische enthalten im allgemeinen bis zu 20 Gew.-% 2,3-Dihalogen-3-sulfonyl-acrylnitrile, bevorzugt 1 bis 10 Gew.-% (bezogen auf 2-Halogen-3-sulfonyl-acrylnitril).

Die mikrobizide Wirkung der beiden Komponenten im Gemisch ist grösser als die der Einzelverbindungen.

Technische Materialien, die durch die erfindungsgemässen Wirkstoffe vor einer mikrobiellen Veränderung und Zerstörung geschützt werden sollen, sind beispielsweise Klebstoffe, Leime, Papiere und Kartons, Textilien, Leder, Holz, Anstrichmittel, Baustoffe, Kautschuk und Kunststoffartikel, Materialien zur Raumdekoration wie Tapeten und Vorhänge, Kühlschmiermittel und Bohröle, die durch Mikroorganismen zersetzt werden können. Im Rahmen der zu schützenden Materialien seien auch Teile von Produktionsanlagen, beispielsweise Kühlwasserkreisläufe, genannt, die durch Mikroorganismen beeinträchtigt werden können.

Mikroorganismen, die einen Abbau oder eine Veränderung der technischen Materialien bewirken können, sind beispielsweise Bakterien, Pilze, Hefen, Algen, Schleime und Viren. Vorzugsweise wirkt das erfindungsgemässe Wirkstoffgemisch aus 2-Halogen-3-sulfonyl-acrylnitril und 2,3-Dihalogen-3-sulfonyl-acrylnitril gegen Pilze und Bakterien.

Es seien beispielsweise Mikroorganismen der folgenden Gattungen genannt:

Escherichia, wie Escherichia coli
Pseudomonas, wie Pseudomonas aeroginosa
Staphylococcus, wie Staphylococcus aureus
Alternaria, wie Alternaria tenues
Aspergillus, wie Aspergillus niger
Chaetomium, wie Chaetomium globosum
Coniofora, wie Coniofora cerebella
Lentinus, wie Lentinus tigrinus
Penicillium, wie Penicillium glaucum
Polyporus, wie Polyporus versicolor
Pullularia, wie Pullaria pullulans
Solerofoma, wie Solerofoma pytholofila
Trichoderma, wie Trichoderma viride

Je nach ihrem Anwendungsgebiet können die erfindungsgemässen Wirkstoffe in die üblichen Formulierungen überführt werden, wie Lösungen, Emulsionen, Suspensionen, Pulver, Pasten und Granulate. Diese können in an sich bekannter Weise hergestellt werden, z.B. durch Vermischen der Wirkstoffe mit einem Streckmittel, aus flüssigem Lösungsmittel und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Wasser als Streckmittel, gegebenenfalls organische Lösungsmittel als Hilfsmittel verwendet werden können.

Flüssige Lösungsmittel oder Wirkstoffe können z.B. Wasser, Alkohole, beispielsweise niedere aliphatische Alkohole, vorzugsweise Ethanol und Isopropanol, araliphatische Alkohole, wie Benzylalkohol, flüssige Kohlenwasserstoffe, wie Benzinfraktionen, chlorierte Kohlenwasserstoffe, wie 1,2-Dichlorethan, Glykole und Polyglykole, Ester wie Ethylacetat, Amylacetat und Butylacetat, Glykolether, wie Methyl- und Phenylglykolether, Ketone, wie Cyclohexanon und Methylethylketon, Säureamide und Säureamidderivate, wie Dimethylformamid und Nitrile, wie Acetonitril, sein.

Feste Trägerstoffe, die bei der Herstellung der fertigen Anwendungsform des Wirkstoffs zugegeben werden, können beispielsweise Talkum, Kieselgur, Bentonit, Kaolin, Diatomenerde und synthetische Gesteinsmehle sein.

Oberflächenaktive Mittel können handelsübliche Emulgatoren, wie Alkylsulfonate, Alkylarylsulfonate, Alkylsulfate, Alkylamidsulfonate, Alkylarylpoly-etheralkohole, Polyoxyethylen-Fettsäureester, oder Dispergiermittel, wie Lignin, Methylcellulose, Polyoxyethylen-Polyoxypropylen, Blockpolymere, sein.

Die Anwendungsform des erfindungsgemässen mikrobiziden Mittels enthält im allgemeinen 1 bis 95 Gew.-%, bevorzugt 5 bis 50 Gew.-% des erfindungsgemässen 2-Halogen-3-sulfonyl-acrylnitrils, vorzugsweise mit 1 bis 10 Gew.-% 2,3-Dihalogen-3-sulfonyl-acrylnitril (Gew.-% bezogen auf das eingesetzte 2-halogen-3-sulfonyl-acrylnitril) als Wirkstoff.

Die Mengen der Anwendungsform des Wirkstoffes bei dem Schutz technischer Materialien können in grösseren Bereichen variiert werden. Im allgemeinen liegen sie im Bereich von 0,001 bis 1 Gew.-%, vorzugsweise im Bereich von 0,001 bis 0,1 Gew.-%, bezogen auf die Gesamtmenge des zu schützenden Materials.

Die erfindungsgemässen Wirkstoffe können in den Formulierungen in Mischungen mit anderen bekannten Wirkstoffen vorliegen. Beispielsweise seien die folgenden Wirkstoffe genannt: Benzimidazolyl-methylcarbamat, Tetramethyl-thiuramidsulfid, N-Fluordichlor-methylthiophthalimid und N,N'-Dimethyl-N'-phenyl-(N-fluor-dichlormethyl-thio)-sulfamid.

Beispiele

Ein 2 l Vierhalskolben ist mit Rührer, Rückflusskühler, Thermometer, pH-Elektrode und zwei 500 ml Tropftrichtern ausgestattet.

Beispiel 1

a) Herstellung von 3-Sulfonyl-propionitril

650 ml Wasser werden in den Kolben gegeben,

auf 80 bis 90°C erhitzt und 935 g (4 Mol) 70 %iges Benzolsulfinsäure-Na-Salz hinzugefügt. Dann wird in ca. 1 Stunde 215 g (4,04 Mol) Acrylnitril und gleichzeitig 382 g (1,95 Mol) 50%ige Schwefelsäure bei 100°C so dosiert, dass der pH-Wert bei 6 bis 7 bleibt. Es wird 30 Minuten bei 100°C nachgerührt und der pH-Wert noch nachreguliert. Überschüssiges Acrylnitril wird über eine Vigreuxkolonne azeotrop mit Wasser bei Normaldruck abdestilliert, dann werden 255 g Xylol zugegeben und bei ca. 95°C die Phasen getrennt. Dazu wird die untere wässrige Phase mit einem Saugrohr abgesaugt. Der Salzniederschlag verbleibt grösstenteils im Kolben. Nun wird bei >95°C 800 ml Waschwasser eingerührt und die Phasen wie oben beschrieben getrennt. Dabei wird der grösste Teil des Salzes gelöst, der Rest stört nicht bei der folgenden Umsetzung. Aus den vereinigten wässrigen Phasen wird noch 17 g 3-Phenylsulfonyl-propionitril isoliert. Zur organischen Phase im Kolben wird nun bei 95 bis 100°C 255 g (300 ml) Xylol gegeben und das Restwasser mit Xylol azeotrop (Kp. 92°C) abdestilliert. Das restliche Xylol wird bei 100 bis 120°C Sumpftemperatur im Vakuum vollständig entfernt. Der Sumpf enthält nun 730 g (94%) 3-Phenylsulfonyl-propionitril in 97%iger Reinheit und 50 g Salz.

b) Umsetzung von 3-Phenyl-sulfonylpropionitril mit $Cl_2$

Ohne Abzukühlen wird nun ein Einleitungsrohr mit Frittenansatz eingeführt, und unter sehr kräftigem Rühren werden bei 110 bis 120°C 531 g (7,47 Mol) Chlor in 6 bis 10 Stunden eingeleitet. Durch analytische DC (Laufmittel Toluol/Essigsäureethylester 9:1) wird der Umsatz geprüft und Chlor (ca. 50 g = 0,7 Mol) nachgeleitet, um durch HCl mitgerissene Chloranteile zu ersetzen. Überschüssiges Chlor und HCl werden nun durch Anlegen von Vakuum bei ca. 110°C in 30 Minuten entfernt. Im Kolben verbleiben 958 g (97%) 2,2-Dichlor-3-phenyl-sulfonyl-propionitril, das ca. 5% 2,3-Dichlor-3-phenyl-sulfonyl-acrylnitril enthält.

c) Umsetzung eines Gemisches von 2,2-Dichlor-Phenyl-sulfonylpropionitril und 2,3-Dichlor-phenylsulfonyl-acrylnitril mit Wasser

Bei ca. 100°C werden 1200 ml Wasser zugegeben. Das Gemisch wird unter Rühren 12 Stunden am Rückfluss gekocht (Sumpftemperatur ca. 120°C), dann bei 90 bis 100°C die (obere) wässrige Phase abgetrennt und die organische Phase bei 90 bis 100°C im Vakuum von Wasserresten befreit. Es werden 820 g (90%) einer hellbraunen Schmelze erhalten, die beim Erkalten wachsartig erhärtet. Durch HPLC wird ein Gehalt von 90 bis 98% 2-Chlor-3-phenylsulfonyl-acrylnitril (cis- und trans-Form) und 2 bis 10% 2,3-Dichlor-3-pheylsulfonylacrylnitril ermittelt.

Beispiele 2 bis 5

In gleicher Weise wie in Beispiel 1 werden die folgenden Verbindungen hergestellt:

| | Schmelzpunkt |
|---|---|
| 2. 2-Chlor-3-(3-chlor-phenyl)-sulfonyl-acrylnitril | 150°C |
| 3. 2-Chlor-3-methyl-sulfonyl-acrylnitril | 53°C |
| 4. 2-Chlor-3-(4-methyl-phenyl)-sulfonyl-acrylnitril | 78°C |
| 5. 2-chlor-3-(3,4-dichlor-phenyl)-sulfonyl-acrylnitril | 107°C |

**Patentansprüche**

1. Verfahren zur Herstellung von 2-Halogen-3-sulfonyl-acrylnitrilen der Formel

$R^1-SO^2-CH=CX^1-CN$,

worin
$R^1$ gegebenenfalls substituiertes Aryl, Aralkyl, Alkyl oder Cycloalkyl und
$X^1$ Halogen bedeutet,
dadurch gekennzeichnet, dass man 2,2-Dihalogen-3-sulfonyl-propionitrile der Formel

$R^1-SO_2-CH_2-CX^1_2-CN$,

worin
$R^1$ $X^1_2$ die angegebene Bedeutung haben, gegebenenfalls in Gegenwart eines organischen Lösungsmittels und gegebenenfalls bei erhöhter Temperatur mit einem Überschuss an Wasser behandelt.

2. Verfahren nach Anspruch 1, dadurch gekennzeichnet, dass die Umsetzung mit Wasser in einem organischen Lösungsmittel im Temperaturbereich von 20 bis 130°C durchgeführt wird.

3. Verfahren nach den Ansprüchen 1, dadurch gekennzeichnet, dass 5 bis 550 Mol Wasser pro Mol 2,2-Dihalogen-3-sulfonyl-propionitril eingesetzt werden.

4. Verfahren nach Anspruch 1, dadurch gekennzeichnet, dass man die Umsetzung mit einem Überschuss an Wasser ohne organisches Lösungsmittel im Temperaturbereich des Siedepunkts des Wassers durchführt.

5. Verfahren nach den Ansprüchen 1 bis 4, dadurch gekennzeichnet, dass die 2,2-Dihalogen-3-sulfonyl-propionitrile durch Halogenierung von 3-Sulfonyl-propionitrilen hergestellt werden.

6. Verfahren nach Anspruch 5, dadurch gekennzeichnet, dass die 3-Sulfonyl-propionitrile durch Umsetzung von Sulfinaten in Wasser, gegebenenfalls in Gegenwart eines organischen Lösungsmittels, mit Acrylnitril unter gleichzeitiger Zugabe von Säure im Temperaturbereich von 50 bis 130°C hergestellt werden.

7. Verfahren zur Herstellung von 2-Halogen-3-sulfonyl-acrylnitrilen, dadurch gekennzeichnet, dass in einem ersten Reaktionsschritt Sulfinate mit Acrylnitril umgesetzt werden, in einem zweiten Reaktionsschritt die erhaltenen 3-Sulfonyl-propionitrile halogeniert werden und die gegebenenfalls im Gemisch mit 2,3-Dihalogen-3-sulfonyl-acrylnitrilen erhaltenen 2,2-Dihalogen-3-sulfonyl-propionitrile mit Wasser umgesetzt werden.

8. Verwendung von 2-Halogen-3-sulfonyl-acrylnitrilen zusammen mit bis zu 20 Gew.-% 2,3-Dihalogen-3-sulfonyl-acrylnitrilen (Gew.-% bezogen auf 2-Halogen-3-sulfonyl-acrylnitril) in mikrobiziden Mitteln.

9. Verwendung von 2-Halogen-3-sulfonyl-acrylnitrilen zusammen mit 1 bis 10 Gew.-% 2,3-Dihalogen-3-sulfonyl-acrylnitrilen (Gew.-% bezogen auf 2-Halogen-3-sulfonyl-acrylnitril), in mikrobiziden Mitteln.

10. Verwendung nach Ansprüchen 8 und 9 zum Schutz technischer Materialien.

**Claims**

1. Process for the preparation of 2-halogeno-3-sulphonyl-acrylonitriles of the formula

$R^1-SO_2-CH=CX^1-CN$

wherein
$R^1$ denotes optionally substituted aryl, aralkyl, alkyl or cycloalkyl and
$X^1$ denotes halogen,
characterised in that 2,2-dihalogeno-3-sulphonyl-propionitriles of the formula

$R^1-SO_2-CH_2-CX^1_2-CN$

wherein
$R^1$ and $X^1$ have the meaning given, are treated with an excess of water, if appropriate in the presence of an organic solvent and, if appropriate, at elevated temperature.

2. Process according to Claim 1, characterised in that the reaction with water is carried out in an organic solvent in the temperature range of from 20 to 130°C.

3. Process according to the Claim 1, characterised in that 5 to 550 mols of water per mol of 2,2-dihalogeno-3-sulphonyl-propionitrile are employed.

4. Process according to Claim 1, characterised in that the reaction is carried out with an excess of water, without organic solvent, in the temperature range of the boiling point of water.

5. Process according to the Claims 1 to 4, characterised in that the 2,2-dihalogeno-3-sulphonyl-propionitriles are prepared by halogenation of 3-sulphonyl-propionitriles.

6. Process according to Claim 5, characterised in that the 3-sulphonyl-propionitriles are prepared by reaction of sulphinates in water, if appropriate in the presence of an organic solvent, with acrylonitrile, with simultaneous addition of acid, in the temperature range of from 50 to 130°C.

7. Process for the preparation of 2-halogeno-3-sulphonyl-acrylonitriles, characterised in that sulphinates are reacted with acrylonitrile in a first reaction step, and in a second reaction step, the 3-sulphonyl-propionitriles obtained are halogenated and the 2,2-dihalogeno-3-sulphonyl-propionitriles, optionally obtained in mixture with 2,3-dihalogeno-3-sulphonyl-acrylonitriles, are reacted with water.

8. Use of 2-halogeno-3-sulphonyl-acrylonitriles, together with up to 20% by weight of 2,3-dihalogeno-3-sulphonyl-acrylonitriles (% by weight relative to 2-halogeno-3-sulphonyl-acrylonitrile) in microbicidal agents.

9. Use of 2-halogeno-3-sulphonyl-acrylonitriles, together with 1 to 10% by weight of 2,3-dihalogeno-3-sulphonyl-acrylonitriles (% by weight relative to 2-halogeno-3-sulphonyl-acrylonitrile), in microbicidal agents.

10. Use according to Claims 8 and 9 for the protection of industrial materials.

**Revendications**

1. Procédé de préparation de 2-halogéno-3-sulfonyl-acrylonitriles de formule:

$R^1-SO_2-CH=CX^1-CN$

dans laquelle
$R^1$ représente un groupe aryle éventuellement substitué, aralkyle, alkyle ou cycloalkyle, et
$X^1$ est un halogène,
procédé caractérisé en ce qu'on traite par un excès d'eau, éventuellement en présence d'un solvant organique et éventuellement à température élevée, des 2,2-dihalogéno-3-sulfonyl-propionitriles de formule:

$R^1-SO_2-CH_2-CX^1_2-CN$

dans laquelle
$R^1$ et $X^1$ ont le sens indiqué.

2. Procédé selon la revendication 1, caractérisé en ce qu'on effectue la réaction avec de l'eau dans un solvant organique dans une gamme de température comprise entre 20 et 130°C.

3. Procédé selon la revendication 1, caractérisé en ce qu'on utilise 5 à 550 moles d'eau par mole de 2,2-dihalogéno-3-sulfonyl-propionitrile.

4. Procédé selon la revendication 1, caractérisé en ce qu'on effectue la réaction avec un excès d'eau sans solvant organique dans une gamme de température correspondant au point d'ébullition de l'eau.

5. Procédé selon les revendications 1 à 4, caractérisé en ce que l'on prépare les 2,2-dihalogéno-3-sulfonyl-propionitriles par halogénation de 3-sulfonyl-propionitriles.

6. Procédé selon la revendication 5, caractérisé en ce qu'on prépare les 3-sulfonyl-propionitriles par réaction de sulfinates dans de l'eau, éventuellement en présence d'un solvant organique, avec l'acrylonitrile, avec addition simultanée d'un acide dans la gamme de températures de 50 à 130°C.

7. Procédé de préparation de 2-halogéno-3-sulfonyl-acrylonitriles, caractérisé en ce qu'on fait réagir dans une première étape de réaction des sulfinates avec l'acrylonitrile, dans une seconde étape de réaction on halogène les 3-sulfonyl-propionitriles obtenus et l'on fait réagir avec l'eau les 2,2-dihalogéno-3-sulfonyl-propionitriles obtenus éventuellement en mélange avec des 2,3-dihalogéno-3-sulfonyl-acrylonitriles.

8. Utilisation de 2-halogéno-3-sulfonyl-acrylonitriles avec jusqu'à 20% en poids de 2,3-dihalogéno-3-sulfonyl-acrylonitriles (en % en poids par rapport au 2-halogéno-3-sulfonyl-acrylonitrile) dans des agents microbicides.

9. Utilisation de 2-halogéno-3-sulfonyl-acrylonitriles avec 1 à 10% en poids de 2,3-dihalogéno-3-sulfonyl-acrylonitriles (en % en poids par rapport au 3-halogéno-3-sulfonyl-acrylonitrile) dans des agents microbicides.

10. Utilisation selon les revendications 8 et 9 pour la protection de matières industrielles ou techniques.